# EUROPÄISCHE PATENTSCHRIFT

(11) **EP 1 359 882 B1**
(45) Veröffentlichungstag und Bekanntmachung des Hinweises auf die Patenterteilung: **18.04.2007**
(21) Anmeldenummer: 02719840.7
(22) Anmeldetag: 14.02.2002
(51) Int. Cl.: A61K 6/06, A61C 13/00

(54) **VERFAHREN ZUR HERSTELLUNG VON ZAHNERSATZ**
METHOD FOR PRODUCING DENTURES
PROCEDE DE REALISATION DE PROTHESE DENTAIRE

(30) Priorität: 14.02.2001 DE 10107451
(43) Veröffentlichungstag der Anmeldung: 12.11.2003
(73) Patentinhaber: 3M ESPE AG, 82229 Seefeld (DE)
(72) Erfinder: FRANK, Sybille, 82229 Seefeld (DE); HAUPTMANN, Holger, 82404 Sindelsdorf (DE); HÖSCHELER, Stefan, 82211 Herrsching (DE); SCHNAGL, Robert, 86899 Landsberg (DE); SUTTOR, Daniel, 82229 Seefeld (DE)
(74) Vertreter: Fiesser, Gerold Michael
(86) Internationale Anmeldenummer: PCT/EP2002/001594
(87) Internationale Veröffentlichungsnummer: WO 2002/064099

(56) Entgegenhaltungen:
- EP-A- 0 624 360
- EP-A- 0 824 897
- WO-A-01/12097
- DE-A- 10 049 974
- DE-A- 19 930 564

## Beschreibung

Die Erfindung betrifft ein Verfahren zur Herstellung von Zahnersatz. Ferner betrifft die Erfindung vorgesinterte Rohlinge aus Zirkonoxidkeramik, die eine Rohbruchfestigkeit in einem ausgewählten Bereich aufweisen.

Keramischer Zahnersatz wird üblicherweise durch Schleifbearbeitung von dichtgesinterten Keramik-Rohlingen hergestellt.

So wird beispielsweise in der EP-B-0 160 797 ein Rohling und dessen Verwendung zur Herstellung zahntechnischer Formteile mittels eines Schleifwerkzeugs beschrieben. Ferner ist aus der EP-A-0 630 622 ein Verfahren zur Herstellung keramischer Dentalprothesen bekannt, bei dem ein Rohling einer bestimmten Zusammensetzung mittels eines rotierenden Werkzeugs schleifend bearbeitet wird.

Nachteilig an der Bearbeitung von dichtgesinterten Rohlingen ist insbesondere deren hohe Härte, die zu langen Bearbeitungszeiten und hoher Werkzeugabnutzung führt. Dadurch sind die Kosten der Bearbeitung dieser Rohlinge sehr hoch.

Nachteilig an Schleifverfahren zur Bearbeitung bzw. Herstellung von keramischem Zahnersatz ist ferner, dass durch das Fehlen von definierten Schneidkanten keine hochpräzise Form der beschliffenen Rohlinge gewährleistet werden kann.

Die Bearbeitung von bis zu einem gewissen Härtegrad vorgesinterten Rohlingen wird in der EP-A-0 630 622 auf Seite 3, Spalte 3, Zeile 13 ff. im Grundsatz erwähnt, wobei aber die Bearbeitung der Rohlinge durch Schleifverfahren beibehalten bleibt.

Vorgesinterte Rohlinge weisen eine niedrigere Härte auf, als Dichtgesinterte und zeigen eine höhere Härte, als Ungesinterte. Es ist daher prinzipiell wünschenswert, um eine leichte Bearbeitung zu gewährleisten bzw. eine Bearbeitung erst zu ermöglichen, vorgesinterte Rohlinge zu verwenden.

Die WO 0112097 A offenbart ein Verfahren zur Herstellung von Zahnersatz unter Verwendung vorgesinterter Rohlinge mit einer Rohbruchfestigkeit im Bereich von 15 bis 30 MPa.

So werden beispielsweise die Bearbeitungswerkzeuge weniger stark abgenutzt, was zu längeren Standzeiten der Werkzeuge und dadurch zu erheblich verringerten Kosten führt. Auch ist die Herstellung feinster Mikrostrukturen erst möglich, indem der vorhersagbare Schrumpf der Keramik beim Dichtsintern zu einer weiteren Verkleinerung der erzeugten Mikrostrukturen führt. Die häufig auftretende mikroskopische Beschädigung der Keramik bei der Bearbeitung ist bei vorgesinterten Rohlingen im Rahmen des Dichtsinterprozesses heilbar.

Um Zahnersatz durch Bearbeiten im nicht-dichtgesinterten Zustand herstellen zu können, wird eine vollkommen homogene Verteilung der Festigkeit und Härte sowie der Dichte innerhalb jeder Raumrichtung des keramischen Rohlings benötigt, die im besonderen auch nach der Vorsinterung des Rohlings erhalten bleibt. Es ist vorteilhaft, Abweichungen in der Dichte- und Härteverteilung der Keramik, wenn filigrane Strukturen oder mehrgliedrige Brücken hergestellt werden sollen, zu vermeiden, da schon geringste Inhomogenitäten zu Sollbruchstellen führen können, die die Haltbarkeit dieser komplexen Strukturen während der Bearbeitung erheblich beeinträchtigen oder zu einem unterschiedlichen Sinterverhalten, welches am Verzug des Werkstückes beim Sintern erkennbar ist, führen können. Ein derartiger Verzug führt jedoch zu schlechter Passgenauigkeit und damit zur Unbrauchbarkeit des Zahnersatzes.

Aus folgenden Gründen hat die Bearbeitung von vorgesinterten Rohlingen bisher nicht zu einer technischen Realisierung geführt:

Die Dichtsinterung eines vorgesinterten Rohlings nach der Bearbeitung geht mit Dimensionsänderungen einher, die schwierig zu berechnen und nur mittels komplizierter Verfahren auf die eigentlichen Fräsparameter zu beaufschlagen sind. Daher sind nachträgliche Korrekturen nach der Dichtsinterung an nichtpassgenauen Zahnersatzteilen notwendig. Diese müssen aufgrund der höheren Härte der dichtgesinterten Zahnersatzteile mittels abtragender Verfahren erfolgen und sind als sehr kritisch zu bewerten, da eine Selbstheilung von Verletzungen der Oberflächenstrukturen, wie sie während des Dichtsinterprozesses stattfindet, nicht mehr nachgeholt werden kann.

Zusammenfassend besteht ein erheblicher Bedarf an Methoden zur Herstellung von passgenauem Zahnersatz durch die Verwendung von vorgesinterten keramischen Rohlingen.

Aufgabe der Erfindung ist es daher, ein verbessertes Verfahren zur Herstellung von passgenauem, hochpräzisem Zahnersatz zur Verfügung zu stellen.

Überraschenderweise kann diese Aufgabe durch ein Verfahren zur Herstellung von Zahnersatz gelöst werden, umfassend die Schritte:
a) Bereitstellung eines Rohlings,
b) Bearbeiten des Rohlings durch fräsende Verfahren,
c) Dichtsintern des Rohlings in einem Temperaturbereich von 1200 bis 1650°C,
wobei der Rohling ein vorgesintertes Material umfasst und eine Rohbruchfestigkeit von 31 bis 50 MPa, bevorzugt 31 bis 40 MPa aufweist.

Unter Rohlingen werden im Rahmen dieser Erfindung nicht bearbeitete Materialblöcke bzw. -presslinge verstanden, die im weiteren durch die Bearbeitung einer Formgebung zugeführt werden. Diese Rohlinge können aus den verschiedensten Materialien, insbesondere Keramik, bestehen.

Unter Zahnersatz sind im Rahmen dieser Erfindung insbesondere Kronen sowie drei- und mehrgliedrige Brücken zu verstehen. Besonders geeignet sind die erfindungsgemäßen Rohlinge zur Herstellung von drei- und mehrgliedrigen Brücken.

Unter Bearbeiten sind im Rahmen dieser Erfindung fräsende Maßnahmen zur Formgebung eines Rohlings zu verstehen, die dazu führen, dass der Rohling in eine dem natürlichen Zahn möglichst nahe kommende Form umgearbeitet wird. Nicht unter Bearbeiten ist die Reinigung des im obigen Sinne bearbeiteten Rohlings oder auch die Entfernung von Stütz- und Haltestrukturen, die aus der Einbettung des Rohlings in eine Rohlingshalterung resultieren, zu verstehen, auch wenn dieses Reinigen durch fräsende Verfahren durchgeführt werden kann.

Die Begriffe "umfassen" und "enthaltend" im Sinne der vorliegenden Erfindung leiten eine nicht-abschließende Aufzählung von Merkmalen ein.

Übliche aus dem Stand der Technik bekannte Rohbruchfestigkeiten für keramische Dentalrohlinge liegen im höheren Festigkeitsbereich, beispielsweise von 75 bis 110 MPa; solche Rohlinge sind nicht für die Erfindung einsetzbar.

Es wurde herausgefunden, dass die Bearbeitung von vorgesinterten Rohlingen, deren Rohbruchfestigkeit außerhalb des erfindungsgemäßen Intervalls liegt, nicht zu brauchbaren Ergebnissen führt. Im Falle von kleineren Rohbruchfestigkeiten resultieren zu weiche Rohlinge, die beim fräsenden Bearbeiten brechen können, im Falle von höheren Rohbruchfestigkeiten erhält man zu harte Rohlinge, die jeweils mit den üblichen Bearbeitungsverfahren nicht bearbeitet werden können.

Die Bearbeitung der erfindungsgemäßen vorgesinterten Rohlinge wird mit fräsenden Verfahren durchgeführt. Durch die äußerst scharfen Schneidkanten der Fräswerkzeuge ist die Erzeugung feinster Mikrostrukturen möglich. Die Schneidkanten des Werkzeuges bleiben über einen langen Benutzungszeitraum scharf, da der Rohling im vorgesinterten Zustand nur eine geringe Härte und Festigkeit aufweist. Bei der fräsenden Bearbeitung des Rohlings arbeitet das Werkzeug der Bearbeitungsmaschine bei der Grobbearbeitung beispielsweise mit einer Drehzahl von 5000 bis 40000 Upm, bevorzugt 15000 bis 25000 Upm bei einer Vorschubgeschwindigkeit von 20 bis 5000 mm/min, bevorzugt 500 bis 3500 mm/min. Die Feinbearbeitung erfolgt beispielweise bei einer Drehzahl von 5000 bis 50000 Upm, bevorzugt 18000 bis 35000 Upm mit einer Vorschubgeschwindigkeit von 20 bis 5000 mm/min, bevorzugt 500 bis 3500 mm/min. Bei beiden Bearbeitungsstufen wird beispielsweise ein Fräserdurchmesser von 0,8 bis 4 mm verwendet.

Besonders bevorzugt werden die Rohlinge ohne eine stützende Struktur, wie sie beispielsweise in der EP-A2-0 824 897, Beispiel beschrieben ist, bearbeitet. Der Bearbeitungsvorgang findet von der mit dem Zahnstumpf in Berührung stehenden und von der mit dem Zahnstumpf nicht in Berührung stehenden Seite des fertig bearbeiteten Zahnersatzteils statt. Es ist hierbei von besonderem Vorteil, dass während des Dichtsintervorgangs der Rohling nicht von einer Hochtemperatureinbettmasse umgeben bzw. gestützt sein muss.

Im Laufe des Dichtsinterprozesses kann der umgearbeitete Rohling mittels Trägervorrichtungen, welche sich an die während des Brennprozesses auftretenden Schwunddimensionen selbständig anpassen, wie sie beispielsweise aus der Patentanmeldung DE-199 04 523 bekannt sind, gehaltert werden, um einen Verzug während des Sinterprozesses zu vermeiden.

Die Rohlinge können aus üblichen Dentalkeramiken bestehen. Unter Dentalkeramiken sind im Rahmen dieser Erfindung Zusammensetzungen zu verstehen, die neben den üblichen keramischen Bestandteilen gegebenenfalls auch noch geringe Mengen anderer Bestandteile (Zusätze), wie Sinterhilfsmittel enthalten können. Die Angabe von Rezepturen in Form von Komponenten und Gew.-% bezieht sich stets auf ein Produkt, welches keine Zusätze mehr enthält. Selbstverständlich sind geringe Spuren von Zusätzen, auch in der vor- bzw. endgesinterten Keramik, aus kinetischen, thermodynamischen oder chemischen Gründen möglich und daher auch als im Schutzumfang dieser Erfindung enthalten zu verstehen.

Insbesondere das Vorhandensein von Verunreinigungen fördert die Entstehung von Glasphasen bzw. Glasanteilen. Bevorzugt sind daher auch Rohlinge, die während dem Dichtsintern keine Glasphasen bzw. Glasanteile bilden.

Die erfindungsgemäßen Rohlinge weisen ferner eine bevorzugte Abweichung von der Linearität des Schrumpfes pro Raumrichtung auf, die kleiner als 0,05%, besonders bevorzugt kleiner als 0,01 % ist.

Bevorzugt bestehen die erfindungsgemäßen Rohlinge aus Aluminiumoxid- oder Zirkonoxidkeramik. Besonders bevorzugt ist hierbei die Zirkonoxidkeramik.

Es ist bekannt, dass die Festigkeit von nichtmetallisch-anorganischen Systemen im allgemeinen vom kritischen Spannungsintensitätsfaktor K_{IC} abhängt. Dieser Faktor ist bei amorphen Werkstoffen, beispielsweise Gläsern deutlich niedriger als bei rein kristallinen Systemen (D. Munz/T. Fett: Mechanisches Verhalten keramischer Werkstoffe, Springer-Verlag). Somit sinkt auch die Festigkeit von Keramiken, wenn sich amorphe Phasen an den Korngrenzen bilden. Die erfindungsgemäß bevorzugt einsetzbaren Keramiken weisen daher beispielsweise einen Wert für K_{IC} von 5 bis 10, bevorzugt 8 bis 10, bestimmt nach EN 843 auf.

Überraschenderweise wurde festgestellt, dass Keramiken auf Zirkonoxidbasis mit einem Sinterzusatz von 0,1 bis zu 0,50 Gew.-% mindestens eines der Oxide der Elemente Aluminium, Gallium, Germanium, Indium eine besonders günstige und gleichmäßig verteilte Härte und Festigkeit aufweisen. Sie sind daher besonders zur erfindungsgemäßen Herstellung von komplexem Zahnersatz und filigranen Strukturen geeignet. Von Vorteil ist es hierbei, wenn die Oxide der oben erwähnten Elemente in einer wie oben definierten Menge mit homogener Verteilung zugesetzt werden und diese nicht, wie etwa Verunreinigungen, ungleichmäßig und mit wechselnder Konzentration verteilt sind. Diese homogene Verteilung kann beispielsweise erreicht werden durch Kofällung, wie sie im Ausführungsbeispiel dieser Erfindung beschrieben ist.

Überdies ist eine gleichmäßige Verteilung der während des Vorsinterprozesses gebildeten Partikel von Vorteil. Die Kornform der Partikel ist bevorzugt equiaxial mit einem mittleren Korndurchmesser kleiner 1 µm, besonders bevorzugt kleiner 0,7 µm.

Die für die Erfindung einsetzbaren Rohlinge weisen üblicherweise ein Porenvolumen von 50 bis 65 % auf. Die mittlere Porengröße liegt üblicherweise im Bereich von 3 µm bis 0,1 µm, wobei der Bereich von 2 µm bis 0,2 µm bevorzugt ist.

Im Falle dieser Keramik wird der Vorsinterprozess in einem bevorzugten Temperaturbereich von 850°C bis 1000°C, besonders bevorzugt zwischen 950°C und 995°C durchgeführt, um die erfindungsgemäße Rohbruchfestigkeit zu erzielen. Der Vorsinterprozess wird beispielsweise über einen Zeitraum von 30 h bis 55 h durchgeführt.

Derartige Keramiksysteme weisen bekanntermaßen die Neigung auf, anisotrop zu schrumpfen, haben also einen in die drei Raumrichtungen unterschiedlichen Schrumpf. Da dieser Schrumpf in jeder Raumrichtung in sich linear ist, sind diese Keramiken überraschenderweise zur Herstellung von extrem passgenauem und komplexem Zahnersatz äußerst geeignet.

Die Verwendung von Zirkonoxidkeramiken im medizinischen Bereich ist allgemein bekannt. Reines Zirkonoxid kann allerdings nicht für mechanische Anwendungen verwendet werden, da es beim Abkühlprozess nach dem Sintern sein Volumen durch Modifikationsänderungen zu stark verändert. Durch Zugabe von Magnesium-, Cer- oder Yttriumoxid läßt sich dieser Prozess aber eindämmen. Eine ausführliche Diskussion findet sich in "Aluminium- und Zirkonoxidkeramik in der Medizin", Sonderdruck aus Industrie Diamanten Rundschau, IDR 2/1993 sowie in der EP-A-0 634 149.

Der Zusatz von 0,1 bis zu 0,50 Gew.-%, bevorzugt 0,15 bis 0,50 Gew.-%, besonders bevorzugt 0,20 bis 0,50 Gew.-%, ganz besonders bevorzugt 0,25 bis 0,50 Gew.-%, mindestens einem der Oxide der Elemente Aluminium, Gallium, Germanium, Indium zu derartigen Keramiken führt zur Erniedrigung der Sintertemperatur und Erhöhung der Stabilität und der hydrolytischen Beständigkeit im Gebrauchszustand. Dieser Sachverhalt findet sich für das Oxid des Aluminiums in der Produktinformation der Firma Tosoh "Zirconia Powder" 09/97 wieder. Die Keramik eignet sich allerdings nicht zur Herstellung von passgenauem Zahnersatz gemäß vorliegender Erfindung, da ohne Einhaltung der erfindungsgemäßen Rohbruchfestigkeit eine fräsende Bearbeitung zu hochpräzisem Zahnersatz aufgrund der vorher diskutierten Effekte nicht möglich ist.

Ebenfalls Gegenstand der vorliegenden Erfindung ist ein vorgesinterter Rohling aus Zirkonoxidkeramik der Zusammensetzung (1), enthaltend:
(A) 91 bis 98,45 Gew.-%, bevorzugt 91 bis 97,25 Gew.-% Zirkonoxid,
(B) 0 bis 3,5 Gew.-%, bevorzugt 0 bis 2,5 Gew.-% Hafniumoxid,
(C) 1,5 bis 6,0 Gew.-%, bevorzugt 2,5 bis 6,0 Gew.-% Yttriumoxid,
(D) 0,05 bis 0,50 Gew.-%, bevorzugt 0,15 bis 0,50 Gew.-%, besonders bevorzugt 0,20 bis 0,50 Gew.-%, ganz besonders bevorzugt 0,25 bis 0,50 Gew.-% mindestens eines der Oxide der Elemente Aluminium, Gallium, Germanium, Indium,
(E) 0 bis 1,9 Gew.-%, bevorzugt 0,0005 bis 1,5 Gew.-% färbende Zusätze,
wobei sich die Gew.-% zu 100 ergänzen müssen und der Rohling eine Rohbruchfestigkeit von 31 bis 50 MPa, bevorzugt 31 bis 40 MPa aufweist.

Unter Komponente (E) der Zusammensetzung (1) sind färbende Oxide aus Elementen der Gruppe Pr, Er, Fe, Co, Ni, Ti, V, Cr, Cu, Mn zu verstehen, wobei bevorzugt Fe₂O₃, Er₂O₃ oder MnO₂ eingesetzt werden.

Ferner ist Gegenstand der Erfindung ein Verfahren zur Herstellung von keramischem Zahnersatz, wobei ein Rohling der Zusammensetzung (1) durch geeignete Bearbeitungsmaßnahmen in ein schwindungsangepasstes, vergrößertes Modell des endgültigen Zahnersatzes umgearbeitet wird und anschließend zu seinen Enddimensionen dichtgesintert wird. Unter schwindungsangepasstem Modell ist ein entsprechend einem Teil des theoretisch erwarteten Schrumpfes vergrößertes Modell des gewünschten Zahnersatzes zu verstehen.

Die technische Herstellung der Zusammensetzung (1) gelingt durch Auflösen der in käuflichem Zirkonsand enthaltenen Komponenten (A) und (B) der Zusammensetzung (1) mit HCl, mechanischer Abtrennung der schwerlöslichen Verunreinigungen und Vereinigung mit den nach Behandlung mit HCl ebenfalls als Oxichloride bzw. Chloride vorliegenden Additiven (C) und (D) als wäßrige, stark saure Lösung.

Färbend wirkende Zusätze gemäß Komponente (E) werden anschließend ebenfalls als Chloride, erhalten durch Auflösung in HCl, zugesetzt.

Es schließt sich eine Kofällung der gelösten Komponenten durch Hydrolyse, Kalzination des Fällungsproduktes, Mahlung des Kalzinates auf die gewünschte Endfeinheit sowie unter Verwendung von temporären Gleit- und Bindemitteln ein Sprühtrockenprozess an.

Das auf diese Weise erhaltene Granulat kann mit bekannten Preßverfahren in die gewünschte Vorform gebracht werden. Diese Presslinge werden durch eine binderabhängige Wärmebehandlung entbindert und bei einer Temperatur zwischen 850°C und 1000°C, vorzugsweise zwischen 950°C und 995°C beispielsweise mit 0,5 h bis 4 h Haltezeit vorgesintert.

Keramikpulver enthaltend die Komponenten (A) bis (D) sind auch käuflich erwerbbar (Fa. Tosoh, Tokyo, Japan).

Die mit gebräuchlichen Verfahren, beispielsweise CAD/CAM oder Kopierfräsen bearbeiteten Rohlinge werden bei 1200°C bis 1650°C, besonders bevorzugt 1350°C bis 1550°C beispielsweise mit 1 bis 3 h Haltezeit dichtgesintert.

Vorzugsweise vor dem Dichtsintern können ästhetische Maßnahmen, wie das individuelle Einfärben, vorgenommen werden. Verwendbar sind beispielsweise Verfahren gemäß der Patentanmeldung DE-199 04 522, wobei die Verwendung ionischer Lösungen mindestens eines der Salze der Seltenen-Erden-Elemente, der Lanthaniden oder der Elemente aus der Gruppe Fe, Co, Ni, Ti, V, Cr, Cu, Mn bevorzugt ist.

Gegebenenfalls werden nach dem Dichtsintern die zu einer Dentalprothese umgearbeiteten Keramikrohlinge aus einer Rohlingshalterung entfernt, wobei beispielsweise eine Halterung aus dem Gebrauchsmuster DE-298 154 86 während der Bearbeitung Anwendung finden kann. Nach der Entfernung aus einer Rohlingshalterung kann sich gegebenenfalls die Nachbearbeitung des Rohlings zum Zwecke der Entfernung von Haltestiften oder Verbindungsstellen zwischen der Rohlingshalterung und dem bearbeiteten Rohling anschließen.

Ferner kann der Rohling durch übliche Maßnahmen verblendet werden. Hierzu kann eine Verblendmasse, die den gleichen Wärmeausdehnungskoeffizient wie der Rohling besitzt, auf den Rohling aufgebrannt werden. Rohlinge, die geeignet sind für die vorliegende Erfindung, können beispielsweise einen Wärmeausdehnungskoeffizient zwischen 9,0 und 10,5 ppm/K, bevorzugt zwischen 9,4 und 9,8 ppm/K aufweisen.

Die Erfindung wird nachfolgend durch Beispiele näher erläutert, ohne dass sie durch diese beschränkt werden soll.

Angaben zu Festigkeiten, insbesondere Bruchfestigkeiten im Rahmen dieser Ausführungen beziehen sich auf den "Punch on three ball Test" gemäß ISO 6872.

Zur Herstellung der erfindungsgemäßen Rohlinge wird von unter Anwendung von Druck erhaltenen Vorkörpern ausgegangen. Bei Herstellung dieser Vorkörper wird beispielsweise von reinen Chloriden, Oxichloriden oder Nitraten ausgegangen, in den Beispielen werden Chloride eingesetzt.

### Herstellungsbeispiele 1 und 2

### Zirkonoxidkeramik mit Aluminiumoxidanteil

Um ca. 200 g fertig dotiertes Pressgranulat zu erhalten, werden die Komponenten gemäß folgender Tabelle in destilliertem Wasser gelöst:

| **Nr.** | **M(ZrCl₄)** [**g]** | **M(YCl₃˙6 H₂O) [g]** | **M(AlCl₃) [g]** | **M(FeCl₃) [g]** | **M(ErCl₃) [g]** |
|---|---|---|---|---|---|
| **1 [Gefärbt]** | 355,6 | 33,4 | 0,65 | 0,77 | 0,29 |
| (%-Anteil als Oxid) | (94,0) | (5,17) | (0,25) | (0,2) | (0,38) |
| **2 [Ungefärbt]** | 357,66 | 33,36 | 0,65 | 0 | 0 |
| (%-Anteil als Oxid) | (94,55) | (5,20) | (0,25) | | |
| | | | | | |
| **Komponente** | (A) | (C) | (D) | (E) | (E) |

Es schließt sich eine Kofällung der gelösten Komponenten durch Hydrolyse an, wobei die vorgenannte Lösung mit 32 l 6-molarer wäßriger NH₄OH-Lösung versetzt wird. Dabei ist ein mindestens 30-facher Überschuß der OH⁻-Konzentration gegenüber dem stöchiometrischen Bedarf empfohlen. Das Fällungsprodukt muss anschließend Cl⁻-frei gewaschen werden. Die Kalzination des Fällungsproduktes erfolgt bei 700°C über 0,75 Stunden, gefolgt von einer Mahlung des Kalzinates auf eine Endfeinheit von D₅₀ = 0,6 µm sowie von einem Sprühtrockenprozess unter Verwendung von temporären Gleit- und Bindemitteln (hier: 2,0 Gew.-% PVA, 0,15 Gew.-% Ölsäure bezogen auf Oxidversatz).

Das erhaltene Granulat wird mit einer isostatischen Presse, beispielsweise bei 1500 bis 2500 bar, bevorzugt 1700 bis 2200 bar in Vorkörper der Abmessungen d = 31 mm und l = 150 mm gebracht.

Die Vorkörper werden durch eine Wärmebehandlung (Aufheizrate: 4 K/min bis 650°C, 1 h Haltezeit) entbindert und bei einer Temperatur bei 970°C mit 0,5 h Haltezeit zu den erfindungsgemäß einsetzbaren Rohlingen vorgesintert.

### Verfahrensbeispiele

Zur Herstellung von passgenauen Brücken werden nach den Herstellungsbeispielen 1 und/oder 2 hergestellten Rohlinge mit einem CAD/CAM-System durch Fräsen bearbeitet und unter den folgenden Parametern dichtgesintert:
Aufheizrate: 10 K/min bis Endtemperatur: 1500°C
Haltezeit bei Endtemperatur: 2 h

Das Ergebnis ist in beiden Fällen ein extrem passgenauer Zahnersatz mit hoher Festigkeit (σ > 1000 MPa).

## Patentansprüche

1. Verfahren zur Herstellung von Zahnersatz, umfassend die Schritte:
a) Bereitstellung eines Rohlings,
b) Bearbeiten des Rohlings durch fräsende Verfahren,
c) Dichtsintern des Rohlings in einem Temperaturbereich von 1200 bis 1650°C,
wobei der Rohling ein vorgesintertes Material umfasst und eine Rohbruchfestigkeit von 31 bis 50 MPa aufweist.

2. Verfahren nach Anspruch 1, wobei der Rohling eine Rohbruchfestigkeit von 31 bis 40 MPa aufweist.

3. Verfahren nach einem der Ansprüche 1 oder 2, wobei bei der fräsenden Bearbeitung des Rohlings das Werkzeug der Bearbeitungsmaschine mit einer Drehzahl von 5000 bis 40000 Upm und einer Vorschubgeschwindigkeit von 20 bis 5000 mm/min bei der Grobbearbeitung und einer Drehzahl von 5000 bis 50000 Upm und einer Vorschubgeschwindigkeit von 20 bis 5000 mm/min bei der Feinbearbeitung sowie jeweils mit einem Fräserdurchmesser von 0,8 bis 4 mm arbeitet.

4. Verfahren nach einem der vorhergehenden Ansprüche, wobei der Rohling von der mit dem Zahnstumpf in Berührung stehenden Seite und von der nicht mit dem Zahnstumpf in Berührung stehenden Seite bearbeitet wird.

5. Verfahren nach einem der vorhergehenden Ansprüche, wobei der vorgesinterte Rohling Zirkonoxid- oder Aluminiumoxidkeramik umfasst.

6. Zahnersatz, herstellbar nach einem Verfahren nach einem der Ansprüche 1 bis 5.

7. Vorgesinterter Rohling aus Zirkonoxidkeramik, enthaltend:
(A) 91 bis 98,45 Gew.-% Zirkonoxid,
(B) 0 bis 3,5 Gew.-% Hafniumoxid,
(C) 1,5 bis 6,0 Gew.-% Yttriumoxid,
(D) 0,05 bis 0,50 Gew.-% mindestens eines der Oxide der Elemente Aluminium, Gallium, Germanium, Indium,
(E) 0 bis 1,9 Gew.-% färbende Zusätze (als Oxide gerechnet),
wobei sich die Gew.-% zu 100 ergänzen müssen und der Rohling eine Rohbruchfestigkeit von 31 bis 50 MPa aufweist.

8. Vorgesinterter Rohling nach Anspruch 7, wobei er
(A) 91 bis 98,35 Gew.-% Zirkonoxid,
(B) 0 bis 2,5 Gew.-% Hafniumoxid,
(C) 1,5 bis 6,0 Gew.-% Yttriumoxid,
(D) 0,15 bis 0,50 Gew.-% mindestens eines der Oxide der Elemente Aluminium, Gallium, Germanium, Indium,
(E) 0 bis 1,9 Gew.-% färbende Zusätze
enthält, wobei sich die Gew.-% zu 100 ergänzen müssen.

9. Vorgesinterter Rohling nach Anspruch 7, wobei er
(A) 91 bis 98,45 Gew.% Zirkonoxid,
(B) 0 bis 3,5 Gew.-% Hafniumoxid,
(C) 1,5 bis 6,0 Gew.-% Yttriumoxid,
(D) 0,05 bis 0,50 Gew.-% Aluminiumoxid,
(E) 0 bis 1,9 Gew.-% färbende Zusätze
enthält, wobei sich die Gew.% zu 100 ergänzen müssen.

10. Vorgesinterter Rohling gemäß einem der Ansprüche 7 bis 9, wobei er eine Rohbruchfestigkeit von 31 bis 50 MPa aufweist.

11. Vorgesinterter Rohling nach einem der Ansprüche 7 bis 10, wobei er durch Sinterung bei einer Temperatur von 850°C bis 1000°C erhalten wird.

12. Vorgesinterter Rohling nach einem der Ansprüche 7 bis 11, wobei er eine Abweichung von der Linearität des Schrumpfes pro Raumrichtung unter 0,05% aufweist.

13. Verwendung eines Rohlings, herstellbar durch ein Verfahren gemäss einem der Ansprüche 1 bis 5 oder eines Rohlings gemäss einem der Ansprüche 7 bis 12 aus vorgesintertem Material mit einer Rohbruchfestigkeit von 31 bis 50 MPa in einem Verfahren zur Herstellung von Zahnersatz.

14. Verfahren zur Herstellung von Zahnersatz nach einem der Ansprüche 1 bis 5, wobei ein Rohling nach einem der Ansprüche 7 bis 12 durch fräsende Bearbeitung in ein schwindungsangepasstes, vergrößertes Modell des endgültigen Zahnersatzes umgearbeitet und zu seinen Enddimensionen dichtgesintert wird.

15. Verfahren zur Herstellung von Zahnersatz nach einem der Ansprüche 1 bis 5, wobei ein Rohling nach einem der Ansprüche 7 bis 12 durch CAD/CAM-Verfahren in ein schwindungsangepasstes, vergrößertes Modell des endgültigen Zahnersatzes umgearbeitet und zu seinen Enddimensionen dichtgesintert wird.

16. Verfahren nach einem der Ansprüche 14 oder 15, wobei der vorgesinterte Rohling nach dem Bearbeiten ästhetisch nachbearbeitet und zu seinen Enddimensionen dichtgesintert wird.

## Claims

1. Method for manufacturing a dental prosthesis, comprising the steps:
a) providing a blank,
b) machining of the blank by milling methods,
c) dense sintering of the blank in a temperature range from 1200 to 1650°C,
in which the blank comprises a presintered material and has a raw breaking strength from 31 to 50 MPa.

2. Method according to claim 1, in which the blank has a raw breaking strength from 31 to 40 MPa.

3. Method according to one of claims 1 or 2, in which during the milling of the blank the tool of the machine works with a rotational speed from 5000 to 40000 rpm and an advance speed from 20 to 5000 mm/min during coarse machining and a rotational speed from 5000 to 50000 rpm and an advance speed from 20 to 5000 mm/min during fine machining, in each case with a milling cutter diameter from 0.8 to 4 mm.

4. Method according to one of the preceding claims, in which the blank is machined from the side in contact with the tooth stump and from the side not in contact with the tooth stump.

5. Method according to one of the preceding claims, in which the presintered blank comprises zirconium oxide ceramic material or aluminium oxide ceramic material.

6. Dental prosthesis which can be manufactured by a method according to one of claims 1 to 5.

7. Presintered blank made of zirconium oxide ceramic material containing:
(A) 91 to 98.45 wt % of zirconium oxide,
(B) 0 to 3.5 wt % of hafnium oxide,
(C) 1.5 to 6.0 wt % of yttrium oxide,
(D) 0.05 to 0.50 wt % of at least one of the oxides of the elements aluminium, gallium, germanium, indium
(E) 0 to 1.9 wt % of colouring additives (calculated as oxides),
in which the wt % must total 100 and the blank has a raw breaking strength from 31 to 50 MPa.

8. Presintered blank according to claim 7, in which it contains
(A) 91 to 98.35 wt % of zirconium oxide,
(B) 0 to 2.5 wt % of hafnium oxide,
(C) 1.5 to 6.0 wt % of yttrium oxide,
(D) 0.15 to 0.50 wt % of at least one of the oxides of the elements aluminium, gallium, germanium, indium,
(E) 0 to 1.9 wt % of colouring additives,
in which the wt % must total 100.

9. Presintered blank according to claim 7, in which it contains
(A) 91 to 98.45 wt % of zirconium oxide,
(B) 0 to 3.5 wt % of hafnium oxide,
(C) 1.5 to 6.0 wt % of yttrium oxide,
(D) 0.05 to 0.50 wt % of aluminium oxide,
(E) 0 to 1.9 wt % of colouring additives,
in which the wt % must total 100.

10. Presintered blank according to one of claims 7 to 9, which has a raw breaking strength from 31 to 50 MPa.

11. Presintered blank according to one of claims 7 to 10, which is obtained by sintering at a temperature from 850°C to 1000°C.

12. Presintered blank according to one of claims 7 to 11, in which it has a deviation from the linearity of the shrinkage in each spatial direction of under 0.05.

13. Use of a blank which can be manufactured by a method according to one of claims 1 to 5 or of a blank according to one of claims 7 to 12 made of presintered material with a raw breaking strength from 31 to 50 MPa in a method for manufacturing a dental prosthesis.

14. Method for manufacturing a dental prosthesis according to one of claims 1 to 5, in which a blank according to one of claims 7 to 12 is worked by milling machining to form an enlarged model of the final dental prosthesis adjusted for shrinkage and dense sintered to its final dimensions.

15. Method for manufacturing a dental prosthesis according to one of claims 1 to 5, in which a blank according to one of claims 7 to 12 is worked by CAD/CAM methods to form an enlarged model of the final dental prosthesis adjusted for shrinkage and dense sintered to its final dimensions.

16. Method according to one of claims 14 or 15, in which after the machining the presintered blank is reworked aesthetically and dense sintered to its final dimensions.

## Revendications

1. Procédé de fabrication d'une prothèse dentaire comprenant les étapes suivantes :
a) Fourniture d'une ébauche,
b) Usinage de l'ébauche par des procédés de fraisage,
c) Frittage à densité maximale de l'ébauche dans une plage de température de 1200 à 1650°C,
l'ébauche comportant une matière préalablement frittée et présentant une résistance à la rupture brute de 31 à 50 MPa.

2. Procédé selon la revendication 1, dans lequel l'ébauche présente une résistance à la rupture brute de 31 à 40 MPa.

3. Procédé selon l'une quelconque des revendications 1 ou 2, dans lequel, lors du fraisage de l'ébauche, l'outil de la machine à usiner travaille à une vitesse de rotation de 5000 à 40000 t/min et à une vitesse d'avancement de 20 à 5000 mm/min lors du dégrossissage et à une vitesse de rotation de 5000 à 50000 t/min et à une vitesse d'avancement de 20 à 5000 mm/min lors du finissage et, respectivement, avec un diamètre de fraise de 0,8 à 4 mm.

4. Procédé selon l'une quelconque des revendications précédentes, dans lequel on usine l'ébauche depuis le côté en contact avec le moignon de la dent et depuis le côté qui n'est pas en contact avec le moignon de la dent.

5. Procédé selon l'une quelconque des revendications précédentes, dans lequel l'ébauche préalablement frittée comporte une céramique à base d'oxyde de zirconium ou d'oxyde d'aluminium.

6. Prothèse dentaire, pouvant être fabriquée avec un procédé selon l'une quelconque des revendications 1 à 5.

7. Ebauche préalablement frittée en céramique à base d'oxyde de zirconium, contenant :
(A) de 91 à 98,45 % en poids d'oxyde de zirconium,
(B) de 0 à 3,5 % en poids d'oxyde d'hafnium,
(C) de 1,5 à 6,0 % en poids d'oxyde d'yttrium,
(D) de 0,05 à 0,50 % en poids d'au moins un des oxydes des éléments aluminium, gallium, germanium, indium,
(E) de 0 à 1,9 % en poids d'additifs colorants (comptés comme oxydes),
les pourcentages en poids devant faire, au total, 100 % et l'ébauche ayant une résistance à la rupture brute de 31 à 50 MPa.

8. Ebauche préalablement frittée selon la revendication 7, laquelle contient
(A) de 91 à 98,35 % en poids d'oxyde de zirconium,
(B) de 0 à 2,5 % en poids d'oxyde d'hafnium,
(C) de 1,5 à 6,0 % en poids d'oxyde d'yttrium,
(D) de 0,15 à 0,50 % en poids d'au moins un des oxydes des éléments aluminium, gallium, germanium, indium,
(E) de 0 à 1,9 % en poids d'additifs colorants, les pourcentages en poids devant faire, au total, 100 %.

9. Ebauche préalablement frittée selon la revendication 7, laquelle contient
(A) de 91 à 98,45 % en poids d'oxyde de zirconium,
(B) de 0 à 3,5 % en poids d'oxyde d'hafnium,
(C) de 1,5 à 6,0 % en poids d'oxyde d'yttrium,
(D) de 0,05 à 0,50 % d'oxyde d'aluminium,
(E) de 0 à 1,9 % en poids d'additifs colorants, les pourcentages en poids devant faire, au total, 100.

10. Ebauche préalablement frittée selon l'une quelconque des revendications 7 à 9, laquelle a une résistance à la rupture brute de 31 à 50 MPa.

11. Ebauche préalablement frittée selon l'une quelconque des revendications 7 à 10, laquelle est obtenue par frittage à une température de 850 à 1000°C.

12. Ebauche préalablement frittée selon l'une quelconque des revendications 7 à 11, dont l'écart par rapport à la linéarité du retrait est inférieur à 0,05% dans toutes les directions spatiales.

13. Utilisation d'une ébauche, pouvant être fabriquée grâce à un procédé selon l'une quelconque des revendications 1 à 5 ou d'une ébauche selon l'une quelconque des revendications 7 à 12, dans une matière préalablement frittée ayant une résistance à la rupture brute de 31 à 50 MPa, dans un procédé de fabrication d'une prothèse dentaire.

14. Procédé de fabrication d'une prothèse dentaire selon l'une quelconque des revendications 1 à 5, dans lequel on transforme une ébauche selon l'une quelconque des revendications 7 à 12, par fraisage, en un modèle de prothèse dentaire définitive agrandi, adapté au retrait, et mis par frittage à densité maximale à ses dimensions finales.

15. Procédé de fabrication d'une prothèse dentaire selon l'une quelconque des revendications 1 à 5, dans lequel on transforme une ébauche selon l'une quelconque des revendications 7 à 12, avec un procédé CAO/DAO, en un modèle de prothèse dentaire définitive agrandi, adapté au retrait, et mis par frittage à densité maximale à ses dimensions finales.

16. Procédé selon l'une quelconque des revendications 14 ou 15, dans lequel on retouche l'esthétique de l'ébauche préalablement frittée après l'usinage et on la met par frittage à densité maximale à ses dimensions finales.
